# EUROPEAN PATENT SPECIFICATION

(11) **EP 3 762 081 B1**
(45) Date of publication and mention of the grant of the patent: **05.11.2025**
(21) Application number: 18909092.1
(22) Date of filing: 09.03.2018
(51) Int. Cl.: A61M 25/00, A61M 25/10

(54) **INFLATABLE MEDICAL BALLOON WITH CONTINUOUS FIBER**
AUFBLASBARER MEDIZINISCHER BALLON MIT ENDLOSFASER
BALLONNET MÉDICAL GONFLABLE À FIBRE CONTINUE

(43) Date of publication of application: 13.01.2021
(73) Proprietor: C. R. Bard, Inc., Franklin Lakes, NJ 07417 (US)
(72) Inventor: HALL, Justin, San Tan Valley, AZ 85140 (US); STAPLETON, Corey E., Gilbert, AZ 85295 (US); HEGDE, Anant, Hayward, CA 94544 (US)
(74) Representative: Hoffmann Eitle
(86) International application number: PCT/US2018/021750
(87) International publication number: WO 2019/172931

(56) References cited:
- US-A1- 2004 082 965
- US-A1- 2006 085 023
- US-A1- 2010 318 029
- US-A1- 2014 277 002
- US-A1- 2015 133 988
- US-A1- 2016 121 076
- US-A1- 2016 121 076
- US-A1- 2016 136 397

## Description

### BACKGROUND

Fiber based devices and expandable devices, such as balloons, are widely used in medical procedures. In the case of a balloon, it is inserted, typically on the end of a catheter, until the balloon reaches the area of interest. Adding pressure to the balloon causes the balloon to inflate. In one variation of use, the balloon creates a space inside the body when the balloon inflates.

Balloons may be used in the heart valves, including during Balloon Aortic Valvuloplasty (BAV) and Transcatheter Aortic Valve Implantation (TAVI). The balloons can be used to open a stenosed aortic valve. A stenosed valve may have hard calcific lesions which may tend to tear or puncture a balloon. Additionally, a precise inflated balloon diameter may be desired for increased safety and control.

Balloons may be used to move plaque away from the center of a vascular lumen toward the vasculature walls, such as during an angioplasty or a peripheral vasculature procedure. During this procedure, a balloon tipped catheter is placed in a vascular obstruction. As the balloon is inflated, the vessel constriction is dilated, resulting in improved blood flow.

Two basic types of balloons are utilized: One is a high pressure, low-compliance balloon. The other is a lower pressure, high-compliance balloon.

High-compliance medical balloons are often composed of urethane, latex, silicone, PVC, Pebax, and other elastomers. As the pressure in a high-compliant balloon is increased, the balloon dimensions expand. Once the pressure is reduced, the high-compliance medical balloon may return to its original shape, or near its original shape. High-compliance medical balloons can easily expand several times in volume between zero inflation pressure and burst.

Traditional high-compliance medical balloons can be inadequate for many reasons. High-compliance, or highly elastic medical balloons typically cannot reach high pressures because their walls have a low tensile strength and their walls thin out as the balloon expands. In some instances, high-compliance medical balloons provide insufficient force to complete a procedure. Exceeding the rated pressure of a high-compliance medical balloon creates an excessive risk of balloon failure which can lead to serious complications for the patient. Moreover, high-compliance medical balloons also have poor shape control. As a high-compliance medical balloon expands, it may assume a shape dictated mostly by the particulars of the environment inside the patient rather than the clinical goals. In some cases, this can be contrary to what the medical practitioner desires. Many medical procedures are predicated on forming a particular balloon shape reliably. Further, high-compliance medical balloons often suffer from poor puncture and tear resistance.

Low-compliance, high pressure medical balloons substantially retain their shape under comparatively high pressures. PET (polyethylene terephthalate) is the most common material for use in high pressure low-compliance balloons. PET is commonly used for highperformance angioplasty balloons. PET is stronger than other polymers, can be molded into a variety of shapes and can be made very thin (e.g., 5 µm to 50 µm (0.0002 in. to 0.002 in.)), thus giving these balloons a low profile. However, balloons made from PET walls are fragile and prone to tears. When pressed against a hard or sharp surface in the body, such as stenosis, PET balloons have poor puncture resistance. PET is very stiff so balloons made from PET may be difficult to pack or fold into a small diameter and may have poor trackability (i.e., the ability to slide and bend over a guidewire deployed through a tortuous vessel). Further, balloons made from PET, while stronger than most other balloons made from homogenous polymers, may still not be strong enough to hold pressures sufficient to complete certain medical procedures. Additionally, with a large balloon diameter (For example, 20 mm or greater), a PET balloon still has excessive compliance for procedures such as BAV and TAVI. Nylon balloons are an alternative material for low-compliance, high pressure balloons. However, these nylon balloons are typically weaker than PET balloons and so can contain less pressure. Nylon readily absorbs water, which can have an adverse effect on Nylon's material properties in some circumstances. Nylon has improved puncture resistance over PET and is more flexible than PET.

Fiber-reinforced composite balloons are another alternative low-compliance, high pressure medical balloon. Such fiber-reinforced composite balloons can advantageously sustain high pressures, provided precise shape control, and are highly resistant to tear and puncture. The manufacturing process for fiber-reinforced balloons, however, can be complicated and expensive, requiring the application of multiple different layers of fibers in order to achieve the desired support. Often, at least one of these layers consists of a fabric de-convolution pattern layer wrapped around a base balloon. Such forming and wrapping of the fabric pattern layer can be cumbersome, labor and equipment intensive, and time consuming. Further, depending upon the orientation of the fibers, the tear pattern of a fiber-reinforced balloon (sometimes referred to as its "rip" or "rip-stop" properties) upon bursting can result in enhanced difficulties in removing the balloon through a shaft. US 2016/121076 discloses a fiber-reinforced inflatable medical balloon, including a cylindrical central portion. The balloon includes first and second tapered or conical end portions connected to the cylindrical central portion along a central longitudinal axis extending from a first end of the balloon to a second end of the balloon. In one embodiment, the balloon includes a single continuous fiber running substantially parallel to the longitudinal axis along the central portion and radially around a portion of the end portions, or both. The balloon comprises a second fiber extending radially around the central portion of the balloon, which may be part of the single continuous fiber.

Thus, there exists the need to create a fiber-reinforced device, such as a balloon, that can be manufactured quickly and easily while still maintaining its ability to withstand high pressures, provide precise shape control, and have highly controlled tear properties.

### SUMMARY OF THE DISCLOSURE

One object of the invention is thus to create a fiber-reinforced device, such as a balloon, that can be manufactured quickly and easily while still maintaining its ability to withstand high pressures, provide precise shape control, and have highly controlled tear properties. The invention is defined by claims 1, 11. Preferred embodiments are defined in the dependent claims.

In general, in the invention, this object is achieved by a medical apparatus comprising a balloon including a central portion and first and second tapered portions connected to the central portion, the balloon including a longitudinal axis. A single continuous fiber extends longitudinally along the balloon, the single continuous fiber having a first turnaround point in the first tapered portion, a second turnaround point in the second tapered portion, and a third turnaround point in the first tapered portion.

In one embodiment, the single continuous fiber comprises: (a) a first pass running substantially parallel to the longitudinal axis along the central portion to the first turnaround point in the first tapered portion; (b) a second pass running substantially parallel to the longitudinal axis along the central portion to the second turnaround point in the second tapered portion; and (c) a third pass running substantially parallel to the longitudinal axis along the central portion to the third turnaround point in the first tapered portion. In one embodiment, the third turnaround point is spaced from the first turnaround point along the longitudinal axis. A fourth turnaround point in the second tapered portion may be spaced from the first turnaround point along the longitudinal axis. The first turnaround point may be provided adjacent an end of the first tapered portion, adjacent a transition from the central portion to the first tapered portion, or at any point between the two.

An adhesive may be used for adhering the single continuous fiber to an outer surface of the balloon. For example, the adhesive may comprise a hot melt glue. The glue cools during the process of applying the single continuous fiber to the balloon, and thus ensures that the fiber is held in place.

The first and third turnaround points may also be aligned in a circumferential direction on the same portion of the balloon. The first and second turnaround points may be on different portions of the balloon, and offset in a circumferential direction.

A further aspect of the disclosure pertains to a medical apparatus comprising a balloon including a central portion, first and second tapered portions connected to the central portion, and a reduced diameter end portion connected to each of the first and second tapered portions, the balloon having a longitudinal axis. A single continuous fiber extends longitudinally along the central portion and the first and second tapered portions in a repeating manner, but does not extend along or around the reduced diameter end portions of the balloon.

It is preferable to provide an inflatable medical balloon with a single continuous fiber, which has turnarounds only in the tapered or cone sections of the balloon. When any reduced diameter portions or necks are removed, and the balloon is attached to a shaft to form a catheter, the single fiber remains continuous.

In one embodiment, the single continuous fiber comprises: (a) a first pass running substantially parallel to the longitudinal axis along the central portion to a first turnaround point in the first tapered portion; (b) a second pass running substantially parallel to the longitudinal axis along the central portion to a second turnaround point in the second tapered portion, and (c) a third pass running substantially parallel to the longitudinal axis along the central portion to a third turnaround point in the first tapered portion. In one embodiment, the third turnaround point is spaced from the first turnaround point along the longitudinal axis. A fourth turnaround point may also be provided in the second tapered portion and spaced from the first turnaround point along the longitudinal axis. The first turnaround point may be adjacent an end of the first tapered portion, adjacent a transition from the central portion to the first tapered portion, or at any point between the two.

In this embodiment, an adhesive may also be used for adhering the single continuous fiber to an outer surface of the balloon. For example, the adhesive may comprise a hot melt glue. The glue cools during the process of applying the single continuous fiber to the balloon, and thus ensures that the fiber is held in place.

The first and third turnaround points may also be aligned in a circumferential direction on the same portion of the balloon. The first and second turnaround points may be on different portions of the balloon, and offset in a circumferential direction.

Still a further aspect of the disclosure pertains to a medical apparatus comprising a balloon including a central portion, first and second tapered portions connected to the central portion, and end portions, the balloon having a longitudinal axis. A first fiber strand extending generally parallel to the longitudinal axis may have a first turnaround point in the first tapered portion, and a second fiber strand extending generally parallel to the longitudinal axis may have a second turnaround point in the first tapered portion. The first and second turnaround points may be staggered along the longitudinal axis.

In one embodiment, the first and second fiber strands are formed by a single continuous fiber. A third fiber strand may have a third turnaround point in the second tapered portion, and a fourth fiber strand may have a fourth turnaround point in the second tapered portion. The third and fourth turnaround points may be staggered along the longitudinal axis, and the third and fourth fiber strands are formed by a single continuous fiber.

In any of these embodiments, a hoop wind of fiber may optionally extend over the single continuous fiber.

Yet another aspect of the disclosure pertains to a medical apparatus having a catheter shaft and an inflatable balloon connected to the catheter shaft. The balloon has a longitudinal axis and a single continuous fiber having a plurality of passes extending generally parallel to the longitudinal axis.

In one embodiment, the inflatable balloon includes a central portion connected to tapered end portions, and the single continuous fiber has a plurality of turnaround points in each of the tapered end portions. The turnaround points may be staggered or offset along the longitudinal axis.

The disclosure also pertains to a method of forming a medical apparatus. The method comprises applying a single continuous fiber to a balloon including a central portion and first and second tapered portions connected to the central portion, the balloon including a longitudinal axis, the single continuous fiber extending longitudinally along the balloon, the single continuous fiber having a first turnaround point in the first tapered portion, a second turnaround point in the second tapered portion, and a third turnaround point in the first tapered portion.

In one embodiment, the balloon includes reduced diameter necks at the ends of the tapered portions, and the single continuous fiber is applied so as to not extend along or over the reduced diameter necks. The method may further include the step of including the step of cutting the reduced diameter necks without cutting the single continuous fiber. The method may also include applying an adhesive to the single continuous fiber prior to or during the applying step. The applying step for applying the fiber may comprise: (1) extending an applicator generally parallel to the longitudinal axis to apply the single continuous fiber to the balloon in a first pass; (2) moving the applicator circumferentially about the balloon a pre-determined distance to apply the fiber in the circumferential direction; and (3) returning the applicator along the longitudinal axis to form a second pass, thereby creating the turnaround point where the fiber changes direction from the first pass to the second pass. These steps may be repeated as desired or necessary.

The disclosure further relates to a medical apparatus, comprising a balloon including a central portion and first and second tapered portions connected to the central portion, and reduced diameter necks connected to each of the first and second tapered portions, the balloon including a longitudinal axis, and a single continuous fiber extending longitudinally along the balloon, the single continuous fiber having a first turnaround point in the first tapered portion, a second turnaround point in the second tapered portion, and a third turnaround point in the first tapered portion, the single continuous fiber extending only along the central portion and the first and second tapered portions, and not along the reduced diameter necks. A catheter including the medical apparatus is also disclosed, wherein the reduced diameter necks are removed to attach the balloon to a catheter shaft, and the single continuous fiber remains on the central portion and first and second tapered portions of the balloon.

Still a further aspect of the disclosure is a medical balloon having a central portion and first and second tapered portions connected to the central portion, and reduced diameter necks connected to each of the first and second tapered portions, the balloon including a longitudinal axis, and having a fiber applied thereto, wherein the reduced diameter necks are free of fiber.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
Figure 1 shows an inflatable balloon having a single continuous fiber laid down on a base balloon so as to form turnaround points in the tapered portions thereof;
Figure 1A is an enlarged, partially cutaway view of a portion of the Figure 1 balloon;
Figure 2 shows an inflatable balloon having a single continuous fiber laid down on a base balloon so as to form turnaround points in the tapered portions thereof, with at least some of the turnaround points being staggered in a longitudinal direction;
Figure 2A is an enlarged, partially cutaway view of a portion of the Figure 2 balloon;
Figure 3 illustrates a balloon having a single continuous fiber forming turnaround points in the tapered portions, along with a hoop fiber wound circumferentially over the single continuous fiber;
Figure 4 illustrates a balloon similar to that of Figure 1, but illustrating that each pass of the fiber may extend at an angle relative to a longitudinal axis;
Figure 5 illustrates one possible manner of applying a single continuous fiber to form a fiber-reinforced balloon;
Figure 6 is a perspective view of a catheter including a balloon formed according to the disclosed concepts.

### DETAILED DESCRIPTION

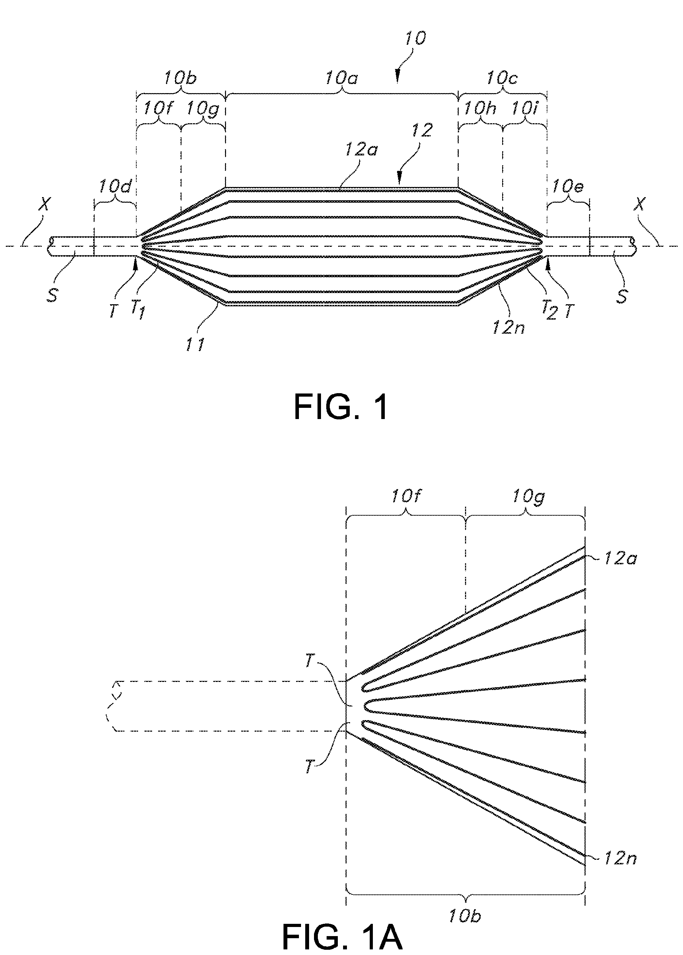

In general, and with reference to Figure 1, described herein is a fiber-reinforced medical balloon 10, which may be a low-compliance, high pressure medical balloon, or a "noncompliant" balloon, either of which generally retains a fixed outer shape and dimension once inflated and regardless of the application of additional internal pressure. The balloon 10 may be formed by the application to an outer surface of a base balloon 11 of a continuous or unbroken substantially inelastic fiber 12 having turnaround points T, as perhaps best understood from the partial view of Figure 1A. By "turnaround point" is it meant that the continuous or unbroken fiber reverses or otherwise changes from going in a first direction, to a generally opposite second direction (but not the directions are not necessarily parallel or even aligned), while remaining continuous at all times during the turning). In other words, the direction of the fiber in a turnaround changes within a range of angles less than 180 degrees, which may comprise, for example, about 140 degrees to 170 degrees. The balloon 10 thus includes multiple passes (e.g., first, second, and third, with every two passes having a turnaround point in common) of fiber strands 12a... 12n extending substantially parallel to the longitudinal axis X within a central portion 10a, with the turnaround points T in the first and second tapered portions 10b, 10c of the balloon 10. However, the turnaround points T stop short of the connected end portions, or necks 10d, 10e, and thus may terminate adjacent to the ends of the tapered portions 10b, 10c (which may each comprise first and second sections 10f; 10g; 10h, 10i, as outlined in more detail in the following description). Thus, the reduced diameter necks are completely free of fiber.

Consequently, the fiber 12 is continuously applied in a single step without cutting from one turnaround point to a different turnaround point while advancing circumferentially, but is not wrapped fully around the balloon 10 in a radial direction. Thus, in one embodiment, the continuous fiber 12 does not intersect with itself in forming the fiber strands 12a ...12n in this manner. Moreover, since the turnaround points T are on the tapered portions 10b, rather than on the necks 10d, 10e, the necks may be removed (as is conventionally done in the process of assembling the balloon 10 into a catheter, along with any sacrificial shaft S on which the balloon 10 is formed) without affecting the continuous nature of the fiber 12 on the balloon 10 itself. That is, in arrangements where a single continuous fiber is wrapped helically around the necks (e.g., as described in U.S. Patent Application Publication No. 2016/0121076), and the necks are trimmed during the manufacturing process, the fibers on the balloon end up being discontinuous as a result of this processing.

Still referring to Figure 1, the fiber 12 may be repeatedly wound back and forth from one end of the balloon 10 to the other end until the fiber strands 12a ... 12n have been laid all the way around the circumference of the central portion 10a. The fiber strands 12a ... 12n can be laid down such that, when the entire circumference is covered, the fiber strands are substantially evenly spaced from one another along the length of the balloon. Thus, as the diameter narrows, the spacing between fiber strands will be reduced in amount, but still stay substantially even from fiber strand to fiber strand.

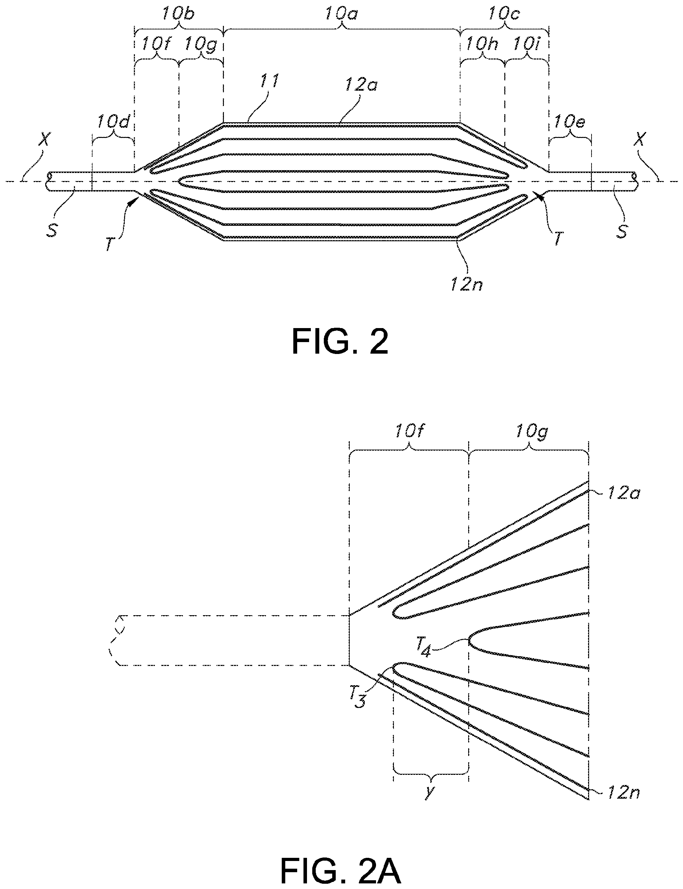

By comparing Figure 1 with Figure 2, it can be understood that the turnaround points T for the various passes may align in the circumferential direction (Figure 1), or instead may be offset from each other (Figure 2). As can be appreciated, the offset means that the turnaround points T are staggered along a longitudinal axis X of the balloon 10 (note offset Y between turnaround points T3, T4 in Figure 2A). In either case, it can be further appreciated that, since each pass of the fiber 12 is along a different portion of the outer surface of the balloon 10, the turnaround points T are also offset in a circumferential direction (that is, one turnaround point T1 on a first tapered portion 10b is not aligned with a turnaround point T2 on a second tapered portion 10c of the balloon 10).

The turnaround points T may also be provided on different portions of the balloon 10. For example, with reference to Figure 1A, it can be understood that the turnaround points T are provided in a first section 10f of the tapered portion 10b, which section is spaced farther away from the central portion 10a. Indeed, each of the turnarounds T may be provided adjacent to the point where the neck 10d is provided (which is typically a vestige of the blow molding process used to form the base balloon 11). In contrast, Figure 2A shows that at least one of the turnaround points T4 is provided in a second section 10g of the tapered portion 10b, while others (including turnaround point T3) are provided in the first section 10f (but closer to the central portion 10a than those in Figure 1). Thus, this turnaround point T4 is actually closer to the transition from the central portion 10a to the tapered portion 10b. It is also possible to provide all turnaround points in the second section 10g of the tapered portion 10b. As can be appreciated, while only tapered portion 10b is shown

It should be understood that while only a relatively small number of fiber passes are shown around the circumference of the central portion 10a for clarity purposes, the pitch can be much higher. For example, the pitch of the fiber strands 12a ... 12n can be between 8 and 100 pitch, more narrowly, 30 and 50 pitch, such as approximately 40 pitch. Further, although the fiber strands were described as being laid down consecutively, they need not be. For example, the strands 12a ... 12n might be laid down in separate groups.

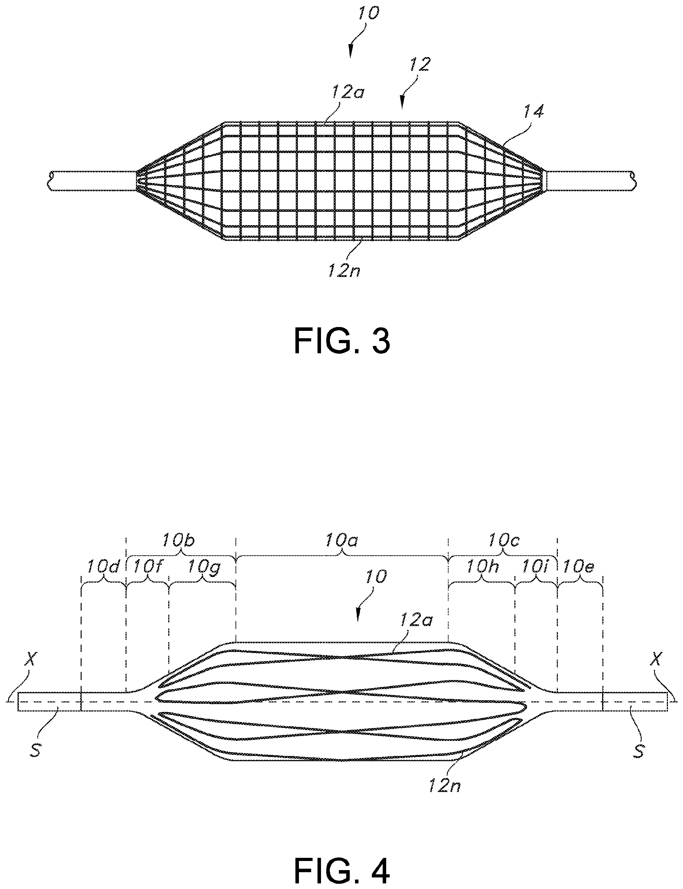

In some embodiments, and with reference to Figure 3, a hoop fiber 14 may be optionally applied over the single continuous fiber 12. In one embodiment, the hoop fiber 14 may be applied over all of the fiber strands 12a ... 12n. Thus, once the fiber strands 12a ... 12n have been formed, the hoop fiber 14 can be wound radially around some or all of the various portions of the balloon 10 to form an overlaying fiber layer. The operation may be repeated as desired, with alternating layers of fiber strands 12a ... 12n and hoop fibers being provided only all or only a portion of the balloon 10.

Referring to Figure 4, in some embodiments, the longitudinal fiber strands 12a ... 12n can extend at an angle relative to the longitudinal axis X within the central portion 10a. For example, the fiber strands 12a ... 12n can extend at an angle of +/- 0 to 20 degrees relative to the longitudinal axis, such as +/-5-15 degrees, such as, for instance, +/-12 degrees. Further, every other fiber strand (or alternating groups of fiber strands) can extend in opposite positive/negative directions relative to the longitudinal axis X, thereby keeping the balloon 10 from twisting. In this embodiment, the fiber strands 12a ... 12n may optionally intersect, as illustrated, or may not (if all fiber strands have the same angular configuration and thus remain substantially parallel).

The fiber 12 described herein can be part of a fiber matrix, such as a plurality of fibers extending within a resin, adhesive, or thermally weldable material (such as a TPU). The resin, adhesive, or thermally weldable material may be applied to the fibers before, during, or after the fibers are placed on the base balloon 11.

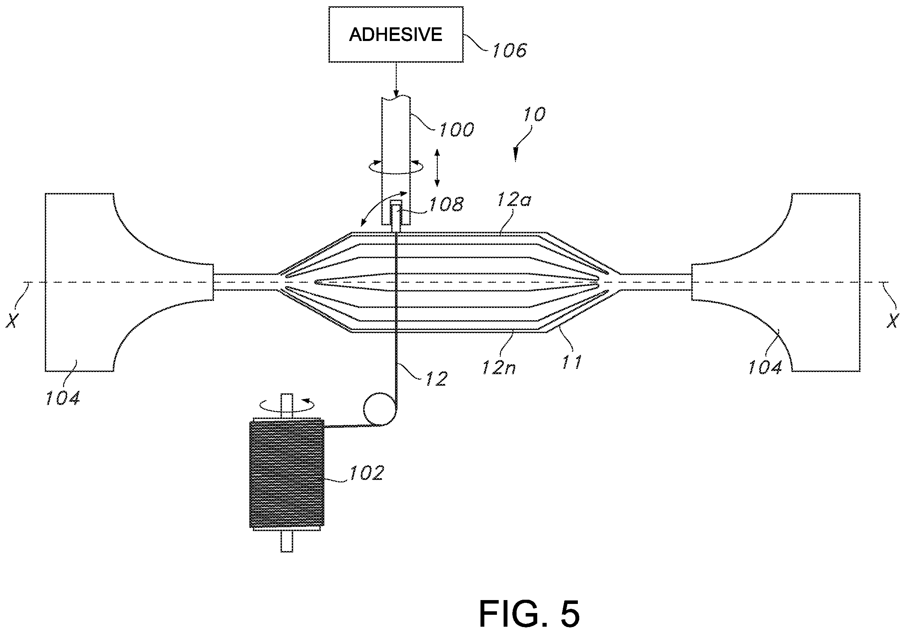

Referring to Figure 5, the fiber 12 can be applied over the base balloon 11 using an automated applicator 100 configured to deliver the fiber from a rotatably mounted spool 102 across the surface of the balloon, which may be held in place from both ends by clamps or holders 104. Before or during the application, the fiber 12 may be infused or coated with an adhesive, a solvent, or both. The applicator 100 can rotate and translate to position the fiber 12 in contact with the base balloon 11. The applicator 100 may apply pressure normal to the surface so as to help attach the fiber 12 to the surface upon which it is being applied and/or spread monofilaments of the fiber tow.

The application of the fiber 12 may involve starting at one tapered end portion 10b, 10c of the base balloon 11, and then extending the applicator 100 generally parallel to the longitudinal axis. When the desired location for forming a turnaround point is reached, the applicator 100 may move circumferentially about the base balloon 11 a pre-determined distance (depending on the desired pitch). The single continuous fiber 12 is thus applied a small distance in the circumferential direction, and then the applicator 100 may return along the longitudinal axis to form a second pass, thereby creating the turnaround point where the fiber changes direction from the first pass to the second pass. This sequence may be repeated as desired and, as can be appreciated, may be modified in the case where the longitudinal passes are to extend at an angle relative to the longitudinal axis X.

In some embodiments, an adhesive or thermally weldable material, such as thermoplastic polyurethane (TPU), can be applied to help stick the fiber 12 to the base balloon 11. The adhesive may be supplied from a reservoir 106 through a nozzle 108 adjacent the point where the fiber 12 exists the applicator 100. In the case of a hot melt glue, both the reservoir 106 and nozzle 108 may be heated to ensure that the flowability remains good.

Further, in some embodiments, the fiber 12 can be dipped through a solvated adhesive or thermally weldable material, such as TPU, during the application. In some embodiments, the material can be applied by spraying. In cases where both solvated thermally weldable material and thermally weldable material are used, the native thermally weldable material can advantageously meet the solvated thermally weldable material to help aid the adhesive properties. Adhesive or thermally weldable material can be applied during application of fiber or after the wind is concluded.

Further, in some embodiments, an outer layer can be applied over the fiber layers. The outer layer can be formed, for example, of a panel or panels of film (not shown) wrapped around the fiber-covered balloon 10.

The fibers described herein can be made from a variety of materials. Exemplary materials include Vectran^{®}, PBO, Spectra^{®}, Conex^{®}, Dyneema^{®}, Technora^{®}, Dacron^{®}, Compet^{®}, Polyester, Nylon, PEEK, PPS, Boron Fiber, Ceramic Fiber, Kevlar^{®}, Inorganic Carbon or Carbon fiber, Inorganic silicon or high strength fiberglass, Organic polymer or aramid, Twaron^{®}, Tungsten, Molybdenum, Stainless Steel, Nickel/cobalt alloys, Titanium alloys, and Nitinol alloys.

Advantageously, the fiber strands 12a ... 12n described herein can be laid down continuously with minimized tooling. The process can be automated and easily updated. The fiber application process can be performed quickly, particularly the application of the strands parallel to the longitudinal axis. Further, since the path of the applicator 100 may be controlled by a computer running software, the automated process allows for ease of changeability between different size and shapes of inflatable balloons. After a base balloon is loaded, the application of all the fiber can be accomplished automatically, with no need for human intervention.

The apparatus described herein can further be engineered to have fiber deposition that exhibits minimized internal wall shear. Wall shear may lead to "slumping" of fiber, wherein fibers, particularly hoop fibers, travel from larger radius sections to smaller radius sections when the balloon is inflated. Travel of hoop fibers may cause premature failure of the balloon and thus limit the balloon's maximum inflation pressure. The fiber strands of the inflatable device described herein further allow for a decreased build-up of fibers at the ends of the balloon relative, for example, to a balloon wound with a helix pattern.

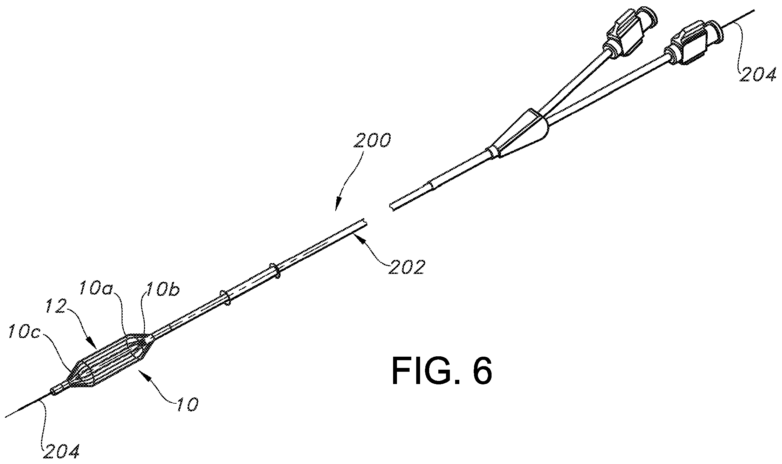

With reference to Figure 6, it can be understood that the balloon 10 incorporating the continuous fiber 12 extending longitudinally along the central portion 10a and with turnarounds in the tapered portions 10b, 10c may be provided as part of a catheter 200. The catheter 200 includes a shaft 202, which may have multiple lumens, such as for providing an inflation fluid to the balloon 10, and accommodating a guidewire 204. As can be appreciated, the single fiber 12 extends only along the central portion 10a and tapered portions 10b, 10c of the balloon 10, and thus remains continuous even after the necks are removed (such as by cutting as a further step during the process of manufacturing the balloon) and the balloon is attached to the shaft 202.

In general, first, second, third etc. turnaround points are referring to different points. Thus, the second turnaround point differs from the first turnaround point, the third turnaround point differs from the first and second turnaround point.

Each of the following terms written in singular grammatical form: "a", "an", and the", as used herein, means "at least one", or "one or more". Use of the phrase One or more" herein does not alter this intended meaning of "a", "an", or "the". Accordingly, the terms "a", "an", and "the", as used herein, may also refer to, and encompass, a plurality of the stated entity or object, unless otherwise specifically defined or stated herein, or, unless the context clearly dictates otherwise. For example, the phrases: "a unit", "a device", "an assembly", "a mechanism", "a component, "an element", and "a step or procedure", as used herein, may also refer to, and encompass, a plurality of units, a plurality of devices, a plurality of assemblies, a plurality of mechanisms, a plurality of components, a plurality of elements, and, a plurality of steps or procedures, respectively.

Each of the following terms: "includes", "including", "has", "having", "comprises", and "comprising", and, their linguistic / grammatical variants, derivatives, or/and conjugates, as used herein, means "including, but not limited to", and is to be taken as specifying the stated component(s), feature(s), characteristic(s), parameter(s), integer(s), or step(s), and does not preclude addition of one or more additional component(s), feature(s), characteristic(s), parameter(s), integer(s), step(s), or groups thereof. Each of these terms is considered equivalent **in meaning** to the phrase "consisting essentially of." Each of the phrases "consisting of and "consists of, as used herein, means "including and limited to". The phrase "consisting essentially of" means that the stated entity or item (system, system unit, system sub-unit device, assembly, sub-assembly, mechanism, structure, component element or, peripheral equipment utility, accessory, or material, method or process, step or procedure, sub-step or sub-procedure), which is an entirety or part of an exemplary embodiment of the disclosed invention, or/and which is used for implementing an exemplary embodiment of the disclosed invention, may include at least one additional feature or characteristic" being a system unit system sub-unit device, assembly, sub-assembly, mechanism, structure, component or element or, peripheral equipment utility, accessory, or material, step or procedure, sub-step or sub-procedure), but only if each such additional feature or characteristic" does not materially alter the basic novel and inventive characteristics or special technical features, of the claimed item.

The term "method", as used herein, refers to steps, procedures, manners, means, or/and techniques, for accomplishing a given task including, but not limited to, those steps, procedures, manners, means, or/and techniques, either known to, or readily developed from known steps, procedures, manners, means, or/and techniques, by practitioners in the relevant field(s) of the disclosed invention.

Terms of approximation, such as the terms about, substantially, approximately, etc., as used herein, refers to ± 10 % of the stated numerical value. Use of the terms parallel or perpendicular are meant to mean approximately meeting this condition, unless otherwise specified.

It is to be fully understood that certain aspects, characteristics, and features, of the invention, which are, for clarity, illustratively described and presented in the context or format of a plurality of separate embodiments, may also be illustratively described and presented in any suitable combination or sub-combination in the context or format of a single embodiment. Conversely, various aspects, characteristics, and features, of the invention which are illustratively described and presented in combination or sub-combination in the context or format of a single embodiment may also be illustratively described and presented in the context or format of a plurality of separate embodiments.

Although the inventions of this disclosure have been illustratively described and presented by way of specific exemplary embodiments, and examples thereof, it is evident that many alternatives thereof, will be apparent to those skilled in the art which fall within the scope of the appended claims.

## Claims

1. A medical apparatus (10), comprising:
a balloon (11) including a central portion (10a) and first (10b) and second (10c) tapered portions connected to the central portion, the balloon including a longitudinal axis (X); and
a single continuous fiber (12) extending longitudinally along the balloon, the single continuous fiber having a first turnaround point (T) in the first tapered portion (10b), a second turnaround point (T) in the second tapered portion (10c), and a third turnaround point (T) in the first tapered portion (10b).

2. The medical apparatus of claim 1,
wherein the single continuous fiber (12) comprises:
a first pass running substantially parallel to the longitudinal axis (X) along the central portion (10a) to the first turnaround point in the first tapered portion (10b),
a second pass running substantially parallel to the longitudinal axis (X) along the central portion (10a) to the second turnaround point in the second tapered portion (10c), and
a third pass running substantially parallel to the longitudinal axis (X) along the central portion (10a) to the third turnaround point in the first tapered portion (10b).

3. The medical apparatus of claim 1 or 2, wherein the third turnaround point is spaced from the first turnaround point along the longitudinal axis (X).

4. The medical apparatus of any of claims 1 to 3, further including a fourth turnaround point in the second tapered portion (10c) and spaced from the first turnaround point along the longitudinal axis (X).

5. The medical apparatus of any of claims 1 to 4, wherein the first turnaround point is adjacent an end of the first tapered portion (10b).

6. The medical apparatus of any of claims 1 to 5, wherein the first turnaround point is adjacent a transition from the central portion (10a) to the first tapered portion (10b).

7. The medical apparatus of any of claims 1 to 6, further including an adhesive adhering the single continuous fiber (12) to an outer surface of the balloon (11).

8. The medical apparatus of claim 7, wherein the adhesive comprises a hot melt glue.

9. The medical apparatus of any of claims 2 to 8, wherein the first and third turnaround points are aligned in a circumferential direction.

10. The medical apparatus of any of claims 1 to 9, wherein the first and second turnaround points are offset in a circumferential direction.

11. A method of forming a medical apparatus, comprising: applying a single continuous fiber (12) to the balloon (11) including a central portion (10a) and first (10b) and second (10c) tapered portions connected to the central portion, the balloon including a longitudinal axis (X), the single continuous fiber (12) extending longitudinally along the balloon, the single continuous fiber having a first turnaround point (T) in the first tapered portion (10b), a second turnaround point (T) in the second tapered portion (10c), and a third turnaround point (T) in the first tapered portion (10b).

12. The method of claim 11, wherein the balloon includes reduced diameter necks at the ends of the tapered portions (10b, 10c), and the single continuous fiber (12) is applied so as to not extend along or over the reduced diameter necks.

13. The method of claim 11, further including the step of cutting the reduced diameter necks without cutting the single continuous fiber (12).

14. The method of claim 11, further including the step of applying an adhesive to the single continuous fiber (12) prior to or during the applying step.

15. The method of claim 11, wherein the applying step comprises:
extending an applicator (100) generally parallel to the longitudinal axis (X) to apply the single continuous fiber (12) to the balloon (11) in a first pass;
moving the applicator (100) circumferentially about the balloon a pre-determined distance to apply the fiber in the circumferential direction;
returning the applicator (100) along the longitudinal axis to form a second pass, thereby creating the turnaround point where the fiber changes direction from the first pass to the second pass.

## Patentansprüche

1. Medizinische Vorrichtung (10), umfassend:
einen Ballon (11), einschließlich eines mittleren Abschnitts (10a) und eines ersten (10b) und eines zweiten (10c) konischen Abschnitts, die mit dem mittleren Abschnitt verbunden sind, wobei der Ballon eine Längsachse (X) einschließt; und
eine einzelne Endlosfaser (12), die sich in der Längsrichtung entlang des Ballons erstreckt, wobei die einzelne Endlosfaser einen ersten Wendepunkt (T) in dem ersten konischen Abschnitt (10b), einen zweiten Wendepunkt (T) in dem zweiten konischen Abschnitt (10c) und einen dritten Wendepunkt (T) in dem ersten konischen Abschnitt (10b) aufweist.

2. Medizinische Vorrichtung nach Anspruch 1,
wobei die einzelne Endlosfaser (12) umfasst:
einen ersten Einzug, der im Wesentlichen parallel zu der Längsachse (X) entlang des mittleren Abschnitts (10a) zu dem ersten Wendepunkt in dem ersten konischen Abschnitt (10b) verläuft,
einen zweiten Einzug, der im Wesentlichen parallel zu der Längsachse (X) entlang des mittleren Abschnitts (10a) zu dem zweiten Wendepunkt in dem zweiten konischen Abschnitt (10c) verläuft, und
einen dritten Einzug, der im Wesentlichen parallel zu der Längsachse (X) entlang des mittleren Abschnitts (10a) zu dem dritten Wendepunkt in dem ersten konischen Abschnitt (10b) verläuft.

3. Medizinische Vorrichtung nach Anspruch 1 oder 2, wobei der dritte Wendepunkt von dem ersten Wendepunkt entlang der Längsachse (X) beabstandet ist.

4. Medizinische Vorrichtung nach einem der Ansprüche 1 bis 3, weiter einschließlich eines vierten Wendepunkts in dem zweiten konischen Abschnitt (10c) und beabstandet von dem ersten Wendepunkt entlang der Längsachse (X).

5. Medizinische Vorrichtung nach einem der Ansprüche 1 bis 4, wobei der erste Wendepunkt benachbart zu einem Ende des ersten konischen Abschnitts (10b) ist.

6. Medizinische Vorrichtung nach einem der Ansprüche 1 bis 5, wobei der erste Wendepunkt benachbart zu einem Übergang von dem mittleren Abschnitt (10a) zu dem ersten konischen Abschnitt (10b) ist.

7. Medizinische Vorrichtung nach einem der Ansprüche 1 bis 6, weiter einschließlich eines Klebstoffs, der die einzelne Endlosfaser (12) an eine Außenfläche des Ballons (11) anhaftet.

8. Medizinische Vorrichtung nach Anspruch 7, wobei der Klebstoff einen Heißschmelzklebstoff umfasst.

9. Medizinische Vorrichtung nach einem der Ansprüche 2 bis 8, wobei der erste und der dritte Wendepunkt in einer Umfangsrichtung miteinander ausgerichtet sind.

10. Medizinische Vorrichtung nach einem der Ansprüche 1 bis 9, wobei der erste und der zweite Wendepunkt in einer Umfangsrichtung versetzt sind.

11. Verfahren zum Bilden einer medizinischen Vorrichtung, umfassend: Aufbringen einer einzelnen Endlosfaser (12) auf den Ballon (11), einschließlich eines mittleren Abschnitts (10a) und eines ersten (10b) und eines zweiten (10c) konischen Abschnitts, die mit dem mittleren Abschnitt verbunden sind, wobei der Ballon eine Längsachse (X) einschließt, wobei sich die einzelne Endlosfaser (12) in der Längsrichtung entlang des Ballons erstreckt,
wobei die einzelne Endlosfaser einen ersten Wendepunkt (T) in dem ersten konischen Abschnitt (10b), einen zweiten Wendepunkt (T) in dem zweiten konischen Abschnitt (10c) und einen dritten Wendepunkt (T) in dem ersten konischen Abschnitt (10b) aufweist.

12. Verfahren nach Anspruch 11, wobei der Ballon an den Enden der konischen Abschnitte (10b, 10c) Hälse mit verringertem Durchmesser einschließt und die einzelne Endlosfaser (12) so aufgebracht wird, dass sie sich nicht entlang der oder über die Hälse mit verringertem Durchmesser erstreckt.

13. Verfahren nach Anspruch 11, weiter einschließlich des Schritts eines Schneidens der Hälse mit verringertem Durchmesser ohne Schneiden der einzelnen Endlosfaser (12).

14. Verfahren nach Anspruch 11, weiter einschließlich des Schritts eines Aufbringens eines Klebstoffs auf die einzelne Endlosfaser (12) vor dem oder während des Aufbringschritts.

15. Verfahren nach Anspruch 11, wobei der Aufbringschritt umfasst:
Erstrecken eines Applikators (100) im Allgemeinen parallel zu der Längsachse (X), um die einzelne Endlosfaser (12) auf den Ballon (11) in einem ersten Einzug aufzubringen;
Bewegen des Applikators (100) in der Umfangsrichtung um den Ballon für eine vorbestimmte Strecke, um die Faser in der Umfangsrichtung aufzubringen;
Zurückführen des Applikators (100) entlang der Längsachse, um einen zweiten Einzug zu bilden, wodurch der Wendepunkt erzeugt wird, an dem die Faser die Richtung von dem ersten Einzug zu dem zweiten Einzug ändert.

## Revendications

1. Appareil médical (10), comprenant :
un ballonnet (11) incluant une partie centrale (10a) et des première (10b) et seconde (10c) parties coniques reliées à la partie centrale, le ballonnet incluant un axe longitudinal (X) ; et
une fibre continue unique (12) s'étendant longitudinalement le long du ballonnet, la fibre continue unique présentant un premier point de rotation (T) dans la première partie conique (10b), un deuxième point de rotation (T) dans la seconde partie conique (10c) et un troisième point de rotation (T) dans la première partie conique (10b).

2. Appareil médical selon la revendication 1,
dans lequel la fibre continue unique (12) comprend :
un premier passage s'étendant de manière sensiblement parallèle à l'axe longitudinal (X) le long de la partie centrale (10a) jusqu'au premier point de rotation dans la première partie conique (10b),
un deuxième passage s'étendant de manière sensiblement parallèle à l'axe longitudinal (X) le long de la partie centrale (10a) jusqu'au deuxième point de rotation dans la seconde partie conique (10c), et
un troisième passage s'étendant de manière sensiblement parallèle à l'axe longitudinal (X) le long de la partie centrale (10a) jusqu'au troisième point de rotation dans la première partie conique (10b).

3. Appareil médical selon la revendication 1 ou revendication 2, dans lequel le troisième point de rotation est espacé du premier point de rotation le long de l'axe longitudinal (X).

4. Appareil médical selon l'une quelconque des revendications 1 à 3, incluant en outre un quatrième point de rotation dans la seconde partie conique (10c) et espacé du premier point de rotation le long de l'axe longitudinal (X).

5. Appareil médical selon l'une quelconque des revendications 1 à 4, dans lequel le premier point de rotation est adjacent à une extrémité de la première partie conique (10b).

6. Appareil médical selon l'une quelconque des revendications 1 à 5, dans lequel le premier point de rotation est adjacent à un passage de la partie centrale (10a) à la première partie conique (10b).

7. Appareil médical selon l'une quelconque des revendications 1 à 6, incluant en outre un adhésif faisant adhérer la fibre continue unique (12) à une surface externe du ballonnet (11).

8. Appareil médical selon la revendication 7, dans lequel l'adhésif comprend une colle thermofusible.

9. Appareil médical selon l'une quelconque des revendications 2 à 8, dans lequel les premier et troisième points de rotation sont alignés dans une direction circonférentielle.

10. Appareil médical selon l'une quelconque des revendications 1 à 9, dans lequel les premier et deuxième points de rotation sont décalés dans une direction circonférentielle.

11. Procédé de formation d'un appareil médical, comprenant : l'application d'une fibre continue unique (12) au ballonnet (11) incluant une partie centrale (10a) et des première (10b) et seconde (10c) parties coniques reliées à la partie centrale, le ballonnet incluant un axe longitudinal (X), la fibre continue unique (12) s'étendant longitudinalement le long du ballonnet, la fibre continue unique présentant un premier point de rotation (T) dans la première partie conique (10b), un deuxième point de rotation (T) dans la seconde partie conique (10c) et un troisième point de rotation (T) dans la première partie conique (10b).

12. Procédé selon la revendication 11, dans lequel le ballonnet inclut des cols de diamètre réduit au niveau des extrémités des parties coniques (10b, 10c), et la fibre continue unique (12) est appliquée de manière à ne pas s'étendre le long des cols de diamètre réduit ou sur ces derniers.

13. Procédé selon la revendication 11, incluant en outre l'étape de découpe des cols de diamètre réduit sans découper la fibre continue unique (12).

14. Procédé selon la revendication 11, incluant en outre l'étape d'application d'un adhésif à la fibre continue unique (12) avant ou pendant l'étape d'application.

15. Procédé selon la revendication 11, dans lequel l'étape d'application comprend :
l'extension d'un applicateur (100) de manière globalement parallèle à l'axe longitudinal (X) pour appliquer la fibre continue unique (12) au ballonnet (11) lors d'un premier passage ;
le déplacement de l'applicateur (100) de manière circonférentielle autour du ballonnet sur une distance prédéterminée pour appliquer la fibre dans la direction circonférentielle ;
le retour de l'applicateur (100) le long de l'axe longitudinal pour former un second passage, créant ainsi le point de rotation où la fibre change de direction, du premier passage au second passage.
